# EUROPEAN PATENT SPECIFICATION

(11) **EP 2 705 844 B1**
(45) Date of publication and mention of the grant of the patent: **22.05.2019**
(21) Application number: 12771356.8
(22) Date of filing: 13.04.2012
(51) Int. Cl.: A61K 31/202, A61K 31/07, A61K 31/198, A61K 31/20, A61K 31/355, A61K 31/375, A61K 31/4415, A61K 31/51, A61K 31/525, A61K 31/714, A61K 33/04, A61K 33/30, A61K 38/00, A61P 3/00, A61P 21/00, A23L 2/52, A61K 38/05

(54) **NUTRITIONAL COMPOSITION**
ERNÄHRUNGSZUSAMMENSETZUNG
COMPOSITION NUTRITIONNELLE

(30) Priority: 13.04.2011 JP 2011089626
(43) Date of publication of application: 12.03.2014
(73) Proprietor: AJINOMOTO CO., INC., Chuo-ku Tokyo 104-8315 (JP)
(72) Inventor: MINE, Tomoyuki, Kawasaki-shi Kanagawa 210-8681 (JP); HAYASHI, Naoki, Kawasaki-shi Kanagawa 210-8681 (JP); KON, Itaru, Kawasaki-shi Kanagawa 210-8681 (JP); SAIMA, Kazunori, Kawasaki-shi Kanagawa 210-8681 (JP)
(74) Representative: Mewburn Ellis LLP
(86) International application number: PCT/JP2012/060186
(87) International publication number: WO 2012/141316

(56) References cited:
- WO-A1-2004/026294
- WO-A1-2004/026294
- WO-A1-2007/043870
- WO-A1-2008/103958
- WO-A1-2008/103958
- WO-A1-2009/015879
- WO-A1-2010/064714
- WO-A1-2011/021926
- WO-A2-2006/105112
- WO-A2-2008/018043
- WO-A2-2009/088738
- WO-A2-2009/100241
- JP-A- 2006 304 792
- JP-A- 2010 105 946
- US-A1- 2005 032 898
- G. I. SMITH ET AL: "Dietary omega-3 fatty acid supplementation increases the rate of muscle protein synthesis in older adults: a randomized controlled trial", AMERICAN JOURNAL OF CLINICAL NUTRITION, vol. 93, no. 2, 15 December 2010 (2010-12-15), pages 402-412, XP055170481, ISSN: 0002-9165, DOI: 10.3945/ajcn.110.005611

### SEQUENCE LISTING

<110> Ajinomoto Co., Inc.
<120> nutrition composition
<130> 091818
<150> JP 2011-089626
   <151> 2011-04-13
<160> 4
<170> PatentIn version 3.5
<210> 1
   <211> 20
   <212> DNA
   <213> Artificial Sequence
<220>
   <223> primer
<400> 1
   acacgccata atggcactgg 20
<210> 2
   <211> 19
   <212> DNA
   <213> Artificial Sequence
<220>
   <223> primer
<400> 2
   cagtcttggc agtgcagat 19
<210> 3
   <211> 20
   <212> DNA
   <213> Artificial Sequence
<220>
   <223> primer
<400> 3
   cgcaaaaccc aatcacctaa 20
<210> 4
   <211> 20
   <212> DNA
   <213> Artificial Sequence
<220>
   <223> primer
<400> 4
   ttggggtttg gcattaagag 20

## Description

### Technical Field

The present invention relates to a nutritional composition containing particular amounts of particular amino acids and n-3 fatty acid, for use in the prophylaxis and/or improvement of particular conditions associated with visceral fat increase, decrease in basal energy consumption, and decrease in basal body temperature.

### Background Art

In elderly people, a specific skeletal muscle decrease called sarcopenia occurs. A decrease in the skeletal muscle reduces the amount of activity of elderly people, and the decreased amount of activity causes further loss of muscle strength, thus forming a vicious circle. A decrease in the amount of activity also decreases the basal energy metabolism. This condition is particularly obvious in a bedridden state.

On the other hand, elderly people have a high risk of falling in a low nutrient condition. According to the search of the Social Insurance Institute in Japan, about 40% of hospital patients and about 30% of home-care patients are in a low nutrient condition. Even when energy ingestion is increased in an attempt to improve the nutrient condition, it does not induce recovery of muscle mass. Conversely, it is said to induce fat accumulation and leads to metabolism associated diseases such as insulin resistance, diabetes, hyperlipidemia, and osteoporosis (non-patent document 1).

To deal with the above-mentioned muscle mass decrease, protein ingestion is recommended. In fact, it has been reported that ingestion of 18 g of an essential amino acid mixture increased protein synthesis of the skeletal muscle (non-patent document 2). However, it is not only difficult for elderly people, who have a depressed kidney function due to the aging, to ingest a large amount (as much as 18 g) of amino acid at once, but also risky since it may place a burden on the kidney. To increase the muscle mass in elderly people, therefore, not only general protein supplementation but also ingestion of more efficient composition is necessary.

On the other hand, it is known that ingestion of lysine increases growth and muscle mass in community residents and animals living on corn and wheat (patent document 1). In this case, however, this is because the protein composition of the staple food is not balanced and lysine becomes a limiting amino acid. In most areas where protein is not necessarily obtained largely from corn and wheat, ingestion of lysine alone does not show a clear effect on the growth of the whole body, or an increase in the muscle mass that appears as one manifestation thereof.

Patent document 2 discloses a composition for patients with cachexia and/or anorexia, which contains a mixed oil with a weight ratio of n-6 fatty acid to n-3 fatty acid of 0.1 - 3.0, amino acid containing branched chain amino acid, and antioxidant such as β-carotene, vitamin C, vitamin E, and selenium. In addition, non-patent document 3 reports that carcinoma cachexia model mouse ingested with n-3 fatty acid and a high leucine-containing protein diet shows a decrease in the body weight and an increase in the muscle mass. In the target patients, particular cytokines (TNF-α, IL-6 and the like) due to cachexia in the cancer late stage are involved, and the muscle proteins collapse based on the cytokines. It is assumed that n-3 fatty acid suppresses secretion of the cytokines, and branched chain amino acid such as leucine aids muscle protein synthesis, whereby the collapse of muscle proteins is prevented. In other words, this composition was studied against the condition of carcinoma cachexia wherein cytokine-dominant protein collapse has extremely progressed. However, it is vastly different from the muscle mass decrease in general elderly people due to a decreased amount of activity by being bedridden and the like, and the effect is unknown.

Further compositions containing amino acids and n-3 fatty acids are disclosed in patent documents 3-6.

Patent document 3 discloses a combination of (i) serine, (ii) cysteine, (iii) arginine and (iv) at least one branched amino acid, for use in the therapeutic or prophylactic treatment of an imbalance in the metabolic use of amino acid resources from the body or from nutrition for acute-phase protein synthesis, in a subject having an inflammation or an infection.

Patent document 4 discloses a composition comprising a therapeutic component including: intact protein; and at least one essential amino acid selected from the group consisting of: leucine, isoleucine, valine, lysine, methionine, histidine, phenylalanine, theonine, and tryptophan; and a carrier component comprising a fruit juice.

Patent document 5 discloses a composition comprising a protein fraction, a carbohydrate fraction and a lipid fraction, wherein the protein fraction provides at least 24.0 % (en% - that is % energetic value of the composition), the carbohydrate provides up to 46 en%, the lipid fraction provides up to 30 en% and wherein at least 12 wt.%, based on proteinaceous matter, is leucine. The composition can be used to treat a subject suffering from insulin resistance.

Patent document 6 discloses a pet food composition comprising: about 5 to about 70% protein, about 0.5 to about 1.6% methionine, about 50 to about 200 ppm manganese, about 0.1 to about 0.5% DHA, about 0.1 to about 0.7% EPA, about 1200 to about 7500 ppm choline, about 1000 to about 2000 ppm taurine, about 2.5 to about 6 % linoleic acid, about 1 to about 3% total n-3 fatty acids, about 50 to about 1200 IU/ kg vitamin E, about 50 to about 500 ppm vitamin C, about 50 to about 500 ppm carnitine, and about 2.5 to about 7g lysine/ 1000 kcal.

Although n-3 fatty acid is used as a hypertriglyceridemia improving agent, a single use of n-3 fatty acid has been reported to show no effect on the weight increase of muscle (non-patent documents 4, 5).

While n-3 fatty acid has been reported to increase mRNA expression of an enzyme involved in fat oxidation (non-patent document 6), enhanced energy metabolism causing enhanced synthesis of protein such as muscle has not been confirmed to date.

As mentioned above, as the situation stands, a nutritional composition having an appropriate amino acid ratio, containing n-3 fatty acid, and having a function to maintain or increase the muscle mass of elderly people has not been provided heretofore.

### [Document List]

### [patent documents]

patent document 1: JP-A-2008-539793
patent document 2: JP-A-H11-508282
patent document 3: WO 2011/021926 A1
patent document 4: WO 2009/100241 A2
patent document 5: WO 2007/043870 A1
patent document 6: WO 2008/103958 A1

### [non-patent documents]

non-patent document 1: Clinical Nutrition, 2008, 8: 38-43
non-patent document 2: Am J Clin Nutr, 2003, 78: 250-258
non-patent document 3: Br J Cancer, 2004, 91: 408-412
non-patent document 4: Drugs of the Future, 2010, 35: 41-52
non-patent document 5: J Nutr Biochem, 2006, 17: 1-13
non-patent document 6: J Nutr, 2005, 135: 2503-2508

### SUMMARY OF THE INVENTION

### Problems to be Solved by the Invention

The problems to be solved by the invention is provision of a novel nutritional composition having a preventive and/or improving effect on
certain conditions; specifically decrease in basal energy consumption, decrease in basal body temperature, and specific conditions associated with visceral fat increase.

### Means of Solving the Problems

The present inventors have conducted various studies in an attempt to solve the aforementioned problems and found that a composition containing particular amount(s) of one or more kinds selected from the group consisting of free lysine, dipeptides containing lysine and lysine salts, and a particular amount of n-3 fatty acid can prevent and/or improve a decrease in the muscle mass, can improve hyperglycemia and hyperlipidemia by suppressing obesity and suppressing accumulation of visceral fat, and such improving effect is remarkable particularly in elderly people, which resulted in the completion of the present invention.

Accordingly, the present invention relates to the following [1] - [10].
[1] A nutritional composition for use in the prophylaxis and/or improvement of a condition by decreasing visceral fat or increasing energy consumption, the composition comprising: n-3 fatty acid; one or more kinds selected from the group consisting of free lysine, dipeptides containing lysine and lysine salts; and branched chain amino acids in free form consisting of valine, leucine and isoleucine; wherein
   the content of the total amount of one or more kinds selected from the group consisting of free lysine, dipeptides containing lysine and lysine salts is 0.1 g - 10.0 g per 100 kcal of the composition, and
   the content of the total amount of n-3 fatty acid is 0.17 g - 5.00 g per 100 kcal of the composition, and wherein the n-3 fatty acid comprises eicosapentaenoic acid; wherein the condition is at least one of:
   dyslipidemia associated with visceral fat increase;
   hyperglycemia associated with visceral fat increase;
   fatty liver associated with visceral fat increase;
   deteriorated liver function associated with visceral fat increase;
   decrease in basal energy consumption; and
   decrease in basal body temperature.
[2] A nutritional composition for use according [1], wherein the condition is at least one of: dyslipidemia associated with visceral fat increase; hyperglycemia associated with visceral fat increase; fatty liver associated with visceral fat increase; and deteriorated liver function associated with visceral fat increase;.
[3] A nutritional composition for use according to [1], wherein the condition is at least one of decrease in basal energy consumption and
   decrease in basal body temperature .
[4] The nutritional composition for use according to any one of [1] to [3], wherein the content of the total amount of one or more kinds of branched chain amino acids in a free form selected from the group consisting of valine,
   leucine and isoleucine is 0.1 g - 15 g per 100 kcal of the composition.
[5] The nutritional composition for use according to any one of [1] to [4], wherein the n-3 fatty acid further comprises one or more kinds selected from the group consisting of docosapentaenoic acid and docosahexaenoic acid.
[6] The nutritional composition for use according to any one of [1] to [5] the preceding claims, comprising 10 mg - 3000 mg of eicosapentaenoic acid per 100 kcal of the composition.
[7] The nutritional composition for use according to any one of [1] to [6], comprising one or more kinds selected from the group consisting of
   0.2 mg - 20.0 mg of vitamin B1 per 100 kcal of the composition,
   0.25 mg - 20.0 mg of vitamin B2 per 100 kcal of the composition,
   0.3 mg - 20.0 mg of vitamin B6 per 100 kcal of the composition, and
   0.3 µg - 20.0 µg of vitamin B12 per 100 kcal of the composition.
[8] The nutritional composition for use according to any one of [1] to [7], comprising one or more kinds selected from the group consisting of
   50 µg retinol equivalents - 2500 µg retinol equivalents of vitamin A per 100 kcal of the composition,
   10 mg - 700 mg of vitamin C per 100 kcal of the composition, and
   1 mg - 50 mg of vitamin E based on α-tocopherol per 100 kcal of the composition.
[9] The nutritional composition for use according to any one of [1] to [8], comprising 1 mg - 50 mg of zinc per 100 kcal of the composition and/or 1.0 µg - 50.0 µg of selenium per 100 kcal of the composition.
[10] The nutritional composition for use according to any one [1] to [9], comprising medium chain fatty acid oil at a content of 10 wt% - 65 wt% relative to the total amount of lipid.

### Effect of the Invention

The nutritional composition disclosed herein is effective for increasing muscle mass and/or suppressing a decrease in muscle mass. The nutritional composition of the present invention is effective for increasing energy consumption. The nutritional composition of the present invention is particularly useful for decreasing visceral fat based on the effect of increasing energy consumption by enhanced fat utilization and the like. The composition is used for the prophylaxis and/or improvement of dyslipidemia, hyperglycemia, fatty liver, and deterioration of liver function associated with visceral fat increase. Furthermore, by increasing muscle mass or suppressing a decrease in muscle mass by fat energy supply and the like, the nutritional composition disclosed herein is also useful for the prophylaxis and/or improvement of sarcopenia, chronic obstructive pulmonary disease (COPD), promotion of rehabilitation effect for muscle recovery, improvement of low nutrient condition, improvement of deterioration in locomotorium, prophylaxis and/or improvement of locomotive syndrome, prevention of falling, increasing muscle mass in sports and the like. Moreover, the nutritional composition of the present invention is effective for the prophylaxis and/or improvement of a decrease in basal energy consumption, and prophylaxis and/or improvement of a decrease in basal body temperature. Furthermore, the nutritional composition of the present invention is highly safe, and can be used for a long time without placing an excessive protein load even for elderly people with attenuated kidney function.

### Brief Description of the Drawings

Fig. 1 shows mesenteric fat weight and gastrocnemius muscle weight of groups 1A - 1D of Experimental Example 1.
Fig. 2 shows mesenteric fat weight and gastrocnemius muscle weight of groups 2A - 2D of Experimental Example 2.
Fig. 3 shows respiratory quotient and energy consumption of groups 3A and 3B of Experimental Example 3.
Fig. 4 shows a ratio of DNA amount of mitochondria gene (mtDNA) to that of nuclear coding gene (chDNA) of groups 3A and 3B of Experimental Example 3.

### Description of Embodiments

The nutritional composition of the present invention comprises one or more kinds selected from the group consisting of free lysine, dipeptides containing lysine and lysine salts in a total amount of 0.1 g - 10.0 g per 100 kcal of the composition, and n-3 fatty acid in a total amount of 0.17 g - 5.00 g per 100 kcal of the composition.

The lysine to be used in the present invention is one of the essential amino acids, becomes limiting amino acid when corn, wheat and the like are staple foods, and particularly effectively utilized by being added to a feed for domestic animals as an amino acid contributing to the efficient growth of the animal. In addition, the lysine to be used in the present invention may be in any form as long as it is in a free form, dipeptide form, salt form, or the like (hereinafter the "lysine" in the present specification means a concept encompassing free lysine, dipeptides containing lysine, lysine salts and the like). Since the lysine contained in the nutritional composition of the present invention is in a free form, a dipeptide form, a salt form or the like, the composition can be used for a long time without placing an excessive protein load even for elderly people with attenuated kidney function. While the lysine to be used in the present invention may be any of an L form, a D form and a DL form, an L form is most preferably used. The lysine in various forms can be used alone, or two or more kinds thereof can be used in combination. While the form of lysine in the present invention is not limited, examples of the lysine salts include acid addition salt, salt with base and the like. Examples of the acid to be added to lysine to form a salt include inorganic acids such as hydrogen chloride, hydrogen bromide, sulfuric acid, phosphoric acid; organic acids such as acetic acid, lactic acid, citric acid, tartaric acid, maleic acid, fumaric acid, monomethylsulfuric acid; acidic amino acids such as glutamate, aspartic acid and the like. In addition, examples of the base that forms a salt with lysine include hydroxide or carbohydroxide of metal such as sodium, potassium, calcium, inorganic bases such as ammonia; organic bases such as ethylenediamine, propylenediamine, ethanolamine, monoalkylethanolamine, dialkylethanolamine, diethanolamine, and triethanolamine. More specific examples of the lysine salts include lysine hydrochloride, lysine acetate, lysine glutamate, lysine aspartate and the like. In the present invention, "dipeptide" refers to an amino acid dimer wherein two amino acid molecules are peptide-bonded, and the "dipeptides containing lysine" refers to a dipeptide wherein at least one molecule (preferably 2 molecules) of the two amino acid molecules constituting the dipeptide is lysine. Examples of the dipeptides containing lysine include lysyllysine and the like. Among these lysines, lysine in a salt form is preferably used, and L-lysine hydrochloride, L-lysine acetate and L-lysine glutamate are particularly preferably used, from the experience of use as a food. Moreover, since flavor, pH upon dissolution and the like vary depending on the form, the form of lysine can be appropriately changed according to use.

The above-mentioned content of lysine is 0.1 g - 10.0 g per 100 kcal of the composition and, from the aspect of amino acid nutrition balance, more preferably 0.2 - 5.0 g, further preferably 0.25 - 4.5 g. When the content of lysine is less than 0.1 g per 100 kcal of the composition, a desired effect cannot be expected since it is used for the constitution of body protein or an energy source. Conversely, when the content exceeds 10.0 g, a large amount of a single amino acid is ingested, which is not very preferable for the amino acid balance. In the present specification, the amount of lysine is based on a free form.

While the daily intake of lysine is individually determined depending on the age, sex, body weight, diet condition and the like, it is preferably 20 mg - 400 mg, more preferably 40 mg - 200 mg, per kg human body weight, from the aspect of amino acid nutrition balance. While the maximum tolerable intake of lysine per day has not been clarified, when it is more than 20 g per human kg body weight, a large amount of a single amino acid is ingested, which is not very preferable for the amino acid balance.

The n-3 fatty acid to be used for the nutritional composition of the present invention is an unsaturated fatty acid having a double bond at the third position from the methyl group end of the hydrocarbon chain. Examples thereof include eicosapentaenoic acid, docosahexaenoic acid, α-linolenic acid, docosapentaenoic acid and the like. These may be used alone, or two or more kinds thereof may be used in combination. The n-3 fatty acid must comprise eicosapenaenoic acid.

The above-mentioned n-3 fatty acid is abundantly contained in fats and oils such as fish oil, Japanese basil oil, and flaxseed oil. In the present invention, they can be extracted from these fats and oils and used after purification. It is also possible to use those produced by a chemical synthesis method, a fermentation method and the like, and a commercially available product for food can also be used. As a lipid source, moreover, the aforementioned fats and oils abundantly containing n-3 fatty acid can also be used directly.

The content of the total amount of n-3 fatty acid is
0.17 g - 5.00 g, more preferably 0.18 g - 4.00 g, further preferably 0.19 g - 3.00 g, per 100 kcal of the composition. When the content of the total amount is less than 0.17 g per 100 kcal of the composition, a clear effect cannot be expected. Conversely, a content exceeding 5.00 g in total per 100 kcal of the composition is not very preferable in view of the flavor. As lipid requirement in Japan, intake of not less than 2 g of n-3 fatty acid per day is recommended based on the lipid ingestion state in the past. In the present invention, fats and oils containing n-3 fatty acid may be directly used, or n-3 fatty acid or fats and oils abundantly containing n-3 fatty acid may be mixed with other oil and used, as long as the content of the n-3 fatty acid falls within the aforementioned range.

The n-3 fatty acid to be used for the nutritional composition of the present invention preferably contains one or more kinds selected from the group consisting of
docosapentaenoic acid and
docosahexaenoic acid, as well as said eicosapentaenoic acid. The content of eicosapentaenoic acid is preferably 10 mg - 3000 mg, more preferably 20 mg - 2000 mg, further preferably 50 mg - 1000 mg, per 100 kcal of the composition. In addition, the content of eicosapentaenoic acid relative to the total amount of n-3 fatty acid is generally 1 wt% - 60 wt%, preferably 5 wt% - 40 wt%. As lipid requirement in Japan, intake of not less than 1 g of eicosapentaenoic acid together with docosahexaenoic acid per day is recommended based on the lipid ingestion state in the past, and the upper limit is not particularly set.

The
nutritional composition of the present invention contains branched chain amino acids consisting of valine, leucine and isoleucine. The form of these branched chain amino acids is free form, and optionally also a
protein form, a peptide form, or a salt form. In consideration of a burden on the kidney and in an attempt to not increase the whole amount of protein,
the nutritional composition includes free form valine, leucine and isoleucine. In this case, any of an L form, a D form and a DL form can be used, and an L form is used most preferably. In addition, as the aforementioned branched chain amino acid, any of the amino acids obtained by extraction from animals and plants etc. containing them, and purification thereof, and the amino acids obtained by a chemical synthesis method, a fermentation method or a gene recombination method may be used.

The above-mentioned branched chain amino acids have been found to be more highly effective when contained in not less than about 0.1 g in total per 100 kcal of the composition, and an increased amount of the branched chain amino acids can be used when a stronger effect is expected. In consideration of degradation of the taste, the content thereof per 100 kcal of the composition is preferably 1.0 g - 20 g, more preferably 1.2 g - 12 g, in total. When the content of the total amount of branched chain amino acids exceeds 20 g per 100 kcal of the composition, a large amount of a particular amino acid is ingested, which is not very preferable for the amino acid balance.

The branched chain amino acids to be used for the nutritional composition of the present invention may all be in a single form or two or more kinds of different forms. For example, the branched chain amino acids to be used for the nutritional composition of the present invention may consist solely of branched chain amino acids in a free form, may contain branched chain amino acids in a free form and a branched chain amino acid in a protein form, and the like. In the nutritional composition of the present invention containing branched chain amino acids in a free form, the content of the total amount thereof is preferably 0.1 g - 15 g, more preferably 0.25 - 12 g, per 100 kcal of the composition.

In the present specification, the amount of branched chain amino acids is based on a free form.

In the present invention, the
mixing ratio of isoleucine, leucine and valine is generally isoleucine:leucine:valine = 1.0:1.5 - 3.0:0.5 - 1.5 in a weight ratio. Particularly, the ratio of leucine is preferably increased to about 1.5- to 3-fold relative to valine.

The nutritional composition of the present invention can contain vitamin Bs.

Examples of the vitamin Bs include vitamin B₁ such as thiamine; vitamin B₂ such as riboflavin; vitamin B₆ such as pyridoxine, pyridoxal, pyridoxamine; vitamin B₁₂ such as cyanocobalamin. These may be used alone or two or more kinds thereof may be used in combination. Vitamin B group plays an important role in the metabolism of carbohydrates, protein and lipid. The content of the vitamin B group is preferably 0.2 mg - 20.0 mg for vitamin B₁, 0.25 mg - 20.0 mg for vitamin B₂, 0.3 mg - 20.0 mg for vitamin B₆, and 0.20 µg - 10.0 µg for vitamin B₁₂, per 100 kcal of the composition of the present invention,.

In addition, the nutritional composition of the present invention can be added antioxidant vitamin and antioxidant mineral. Examples of the antioxidant vitamin include vitamin A such as retinol, retinal, retinoic acid; vitamin C such as ascorbic acid; carotenoid such as β-carotene; vitamin E such as α-tocopherol, and the like. Examples of the antioxidant mineral include zinc, selenium and the like. These may be used alone, or two or more kinds thereof may be used in combination. When fat is utilized as an energy source for muscle protein synthesis, since radical is produced and oxidative stress is caused when radical is produced in excess, an antioxidant is preferably added for the prevention and/or improvement thereof. The content of antioxidant vitamin and antioxidant mineral is preferably 50 µg retinol equivalents - 2500 µg retinol equivalents for vitamin A, 10 mg - 700 mg for vitamin C, 1 mg - 50 mg based on α-tocopherol for vitamin E, 1 mg - 50 mg for zinc, and 1.0 µg - 50.0 µg for selenium, per 100 kcal of the composition of the present invention, to afford a sufficient antioxidant action.

The nutritional composition of the present invention can also contain vitamins other than the aforementioned vitamin B group and antioxidant vitamin (e.g., vitamin D, vitamin K, niacin, folic acid, pantothenic acid, biotin etc.), and minerals other than the aforementioned antioxidant mineral (e.g., sodium, calcium, iron, phosphorus, magnesium, potassium, copper, iodine, manganese, chrome, molybdenum etc.). These may be used alone, or two or more kinds thereof may be used in combination.

In addition, the nutritional composition of the present invention can contain a medium chain fatty acid oil as a lipid source. The "medium chain fatty acid" means a fatty acid having a carbon number of preferably 8 to 10 such as caprylic acid, capric acid, and the "medium chain fatty acid oil" means fats and oils including triglyceride of the aforementioned medium chain fatty acid, and the like. The medium chain fatty acid is characterized in that it is digested and absorbed about 4 times more rapidly than long chain fatty acid generally present in fats and oils, delivered after absorption to the liver via portal vein without passing through lymphatic vessels, and rapidly metabolized. Therefore, it can be preferably utilized as an energy source. For the object of the present invention, fats and oils containing a large amount of medium chain fatty acid such as coconut oil, palm oil, palm kernel oil can be used as the medium chain fatty acid oil. As the coconut oil, palm oil and the like, one extracted and purified from natural plants such as coconut may be used. However, a commercially available product is conveniently used. In the present invention, medium chain fatty acid oil is appropriately contained in 10 wt% - 65 wt% relative to the total weight of lipid.

The nutritional composition of the present invention can contain, as a fat source besides medium chain fatty acid oil, for example, edible vegetable oil such as cottonseed oil, sunflower oil, peanut oil, canola oil, soybean oil, safflower oil, olive oil, rice oil, corn oil, benne oil, cacao butter, edible animal oil such as beef fat, lard, fish oil, butter, butter oil, processed fats and oils such as shortening. These may be used alone, or two or more kinds thereof may be used in combination.

The nutritional composition of the present invention can contain, as a carbohydrate source, for example, glucide, dietary fiber and the like. Examples of glucide include dextrin, oligosaccharide, saccharose, glucose, fructose, starch and the like. Examples of dietary fiber include water-soluble dietary fiber (e.g., indigestible dextrin, pectin, galactomannan etc.), insoluble dietary fiber (e.g., soybean- and wheat-derived bran, crystalline cellulose etc.) and the like. These may be used alone, or two or more kinds thereof may be used in combination.

The nutritional composition of the present invention can contain, as a protein source, amino acid, peptide, protein and the like. As the protein, animal-derived protein such as casein, acid casein, casein sodium, casein calcium, whey protein, milk serum whey protein, fish protein, egg protein, and hydrolysates thereof, and plant-derived protein such as soybean protein, wheat protein, corn protein, and hydrolysates thereof, and the like can be added. The content of the total amount of these protein sources in the nutritional composition of the present invention is preferably 2 - 20 g per 100 kcal of the composition of the present invention. When these protein sources contain branched chain amino acid, it is needless to say that the amount of the branched chain amino acid is included, by calculation, in the aforementioned total content of the branched chain amino acids in the composition of the present invention.

When the nutritional composition of the present invention contains a branched chain amino acid in a protein form, the total content of the protein source in the nutritional composition of the present invention also including the protein is preferably 2 - 20 g per 100 kcal of the composition of the present invention. Particularly, when the branched chain amino acids to be used in the nutritional composition of the present invention consists only of branched chain amino acids in a free form, the content of the total amount of the protein source in the nutritional composition of the present invention is preferably 1.0 - 15 g per 100 kcal of the composition of the present invention.

The nutritional composition of the present invention can be directly fed into the bowels and stomach of patients showing insufficient oral ingestion, by using an administration tube, and when oral ingestion is possible, it can be given as a food or drink. In the present invention, lysine and n-3 fatty acid, and the branched chain amino acids in free form consisting of valine, leucine and isoleucine may be directly mixed and ingested as a nutritional composition, or the composition can also be formulated with a pharmaceutically acceptable carrier for pharmaceutical products and provided as a pharmaceutical product. Alternatively, the composition can also be provided as foods and drinks such as food with health claims such as food for specified health uses and food with nutrient function claims, nutrition aid food, the other health food, by adding a material for food and drink or food additive, or can be provided as general food and drink.

When the nutritional composition of the present invention is formulated and provided, it can be produced as a liquid preparation such as elixir, suspension, syrup, emulsion, ampoule; a solid preparation such as gel, gum, drop, powder, granule, pill, tablet (including sugar-coated tablet, film-coated tablet), capsule, package agent, powder, and the like.

Examples of the pharmaceutically acceptable carrier for pharmaceutical products, which can be used for formulating the nutritional composition of the present invention, include cellulose and a derivative thereof such as crystalline cellulose, hydroxypropylcellulose; excipients such as natural polymer compound (gum arabic, sodium alginate etc.); binders such as guar gum, stearic acid, polymeric polyvinylpyrrolidone; lubricants such as talc, polyethylene glycol 6000; disintegrants such as adipic acid, surfactants such as sucrose fatty acid ester, soybean lecithin, polyoxyethylene hydrogenated castor oil, polyoxyethylene monostearic acid ester; thickeners such as sodium carboxymethylcellulose, carboxyvinyl polymer, xanthan gum, gelatin; coating agents such as ethyl acrylate-methyl methacrylate copolymer dispersion, caramel, Carnauba wax, shellac-pullulan; pH adjusters such as citric acid, sodium citrate, acetic acid, sodium acetate, sodium hydroxide; antioxidants such as erythorbic acid, butylhydroxyanisole, propyl gallate; flavoring agents such as aspartame, licorice extract, saccharin; preservatives such as sodium benzoate, sodium edetate, sorbic acid, sodium sorbate, methyl paraoxybenzoate, butyl paraoxybenzoate; colorants such as ferric oxide red, yellow iron oxide, black iron oxide, carmine, Food Color blue No.1, Food Color yellow No. 4, Food Color Red No. 2 and the like.

When the nutritional composition of the present invention can be provided as food and drink, liquid products such as drinks, milk products such as milk, milk beverage, yogurt, jelly products such as jelly drinks, jelly, gum products, powder products, granular products, sheet products, capsule products, tablet products, solid products such as snack bar, cookie, and the like.

Examples of the materials for food and food additives which can be used for forming the nutritional composition of the present invention as foods and drinks include sweetener, colorant, preservative, thickening stabilizer, antioxidant, color former, bleach, fungicide, gum base, bittering agent, enzyme, gloss agent, acidulant, seasoning, emulsifier, enhancement agent, agent for production, flavor, spice and the like.

When the nutritional composition of the present invention is provided as food and drink, a package form for one ingestion amount unit can be employed. The "package form for one ingestion amount unit" is used when the amount of food and drink to be ingested per meal is determined in advance. Examples thereof include a package of an amount to be ingested at one time using a container such as pack, bag, bottle, box in case of drinks, gum, jelly, yogurt, cookie and the like, and an individual package of an amount to be ingested at one time using pack, bag and the like in case of foods in the form of granule, powder, slurry and the like. Particularly, when the foods and drinks are food with health claims such as food for specified health uses, food with nutrient function claims, special-use foods such as nutrition aid food, invalid food, the other health foods and the like, a form wherein the composition of the present invention is packed in a unit amount to be ingested per meal, a form wherein the composition of the present invention is suspended or dissolved to give a drink, which is packed in a bottle etc. for a single consumption and the like can be mentioned.

The amount of the nutritional composition of the present invention to be ingested per day is individually determined depending on the age, sex, body weight, meal condition and the like, and the nutritional composition of the present invention is appropriately ingested to provide about 50 kcal - 2000 kcal for an adult per day. The aforementioned amount is preferably ingested in about 1 to 3 portions a day. When the nutritional composition of the present invention is formed in a food or drink in a package form of one ingestion amount unit, the amount to be ingested one time as determined above is individually packed.

The nutritional composition of the present invention can be produced by conventional formulation techniques and food production techniques.

The nutritional composition of the present disclosure is useful as a pharmaceutical product, food and drink, and the like for increasing muscle mass and/or suppressing a decrease in muscle mass. In addition, the nutritional composition of the present disclosure is useful as a pharmaceutical product, food and drink, and the like for increasing energy consumption. Furthermore, the nutritional composition of the present disclosure is useful as a pharmaceutical product, food and drink, and the like for the prophylaxis and/or improvement of a decrease in muscle mass unaccompanied by an increase in inflammatory cytokine.

The nutritional composition of the present disclosure is useful as a pharmaceutical product, food and drink, and the like for the prevention and/or improvement of muscle weakness symptom. The muscle weakness symptom is also called sarcopenia, decreases the amount of activity due to the decreased muscular strength of four limbs, and further causes chronic obstructive pulmonary diseases (COPD). The nutritional composition of the present invention can effectively prevent and/or improve such muscle weakness symptom.

The nutritional composition of the present disclosure is useful as a pharmaceutical product, food and drink, and the like for enhancing rehabilitation effect for muscle recovery. Recovery of muscular strength by rehabilitation takes time, but the muscle function needs to be recovered before progression of contracture. The composition of the present invention can increase muscle mass and is useful for enhancing rehabilitation effect. It is also useful for the improvement of low nutrient condition, improvement of deterioration in locomotorium, prophylaxis and/or improvement of locomotive syndrome, prevention of falling and the like, as well as increase of muscle mass in sports and the like.

The nutritional composition of the present invention is useful as a pharmaceutical product, food and drink, and the like for decreasing visceral fat. Increase in visceral fat induces insulin resistance, which develops or progresses various diseases associated with insulin resistance. The nutritional composition of the present invention can effectively reduce visceral fat.

The nutritional composition of the present invention is useful as a pharmaceutical product, food and drink and the like for the prophylaxis and/or improvement of dyslipidemia associated with visceral fat increase. When the visceral fat increases, dyslipidemia such as high LDL-cholesterolemia, hypertriglyceridemia, remnant hyperlipoproteinemia, high small dense LDL, and low HDL-cholesterolemia are observed. As a result of the onset of dyslipidemia, the production of lipoperoxides such as LDL cholesterol peroxide increases, and the risk of developing atherosclerosis becomes high. The nutritional composition of the present invention can effectively suppress the onset of dyslipidemia such as increased blood LDL cholesterol associated with visceral fat increase, and is useful for the prevention and/or improvement of dyslipidemia associated with visceral fat increase.

The nutritional composition of the present invention is useful as a pharmaceutical product, food and drink and the like for the prophylaxis and/or improvement of hyperglycemia associated with visceral fat increase. Insulin resistance due to visceral fat increase depresses glucose uptake efficiency from blood into the cell, and causes hyperglycemia. The nutritional composition of the present invention is useful for the prophylaxis and/or improvement of hyperglycemia associated with visceral fat increase.

The nutritional composition of the present invention is useful as a pharmaceutical product, food and drink and the like for the prophylaxis and/or improvement of fatty liver and deterioration of liver function associated with visceral fat increase. Mesenteric fat, which is a representative increased visceral fat, releases free fatty acid into the portal blood, and causes accumulation of fat in the liver, which is the nearest organ. On the other hand, hyperinsulinemia due to insulin resistance also activates synthesis of fat from sugar in the liver. Furthermore, fat accumulation in the liver aggravates liver function. The nutritional composition of the present invention is useful for the prophylaxis and/or improvement of fatty liver and deterioration of liver function associated with visceral fat increase.

The nutritional composition of the present invention is useful as a pharmaceutical product, food and drink and the like for the prophylaxis and/or improvement of a decrease in basal energy consumption. A decrease in the basal energy consumption in elderly people means depressed energy utilization in the body, which exerts a vast influence on the metabolism such as synthesis of protein to be utilized as energy. In addition, when the energy utilization is depressed, the ingested energy becomes redundant, and the redundant energy is accumulated as fat. The nutritional composition of the present invention is useful for the prophylaxis and/or improvement of a decrease in the basal energy consumption.

The nutritional composition of the present invention is useful as a pharmaceutical product, food and drink and the like for the prophylaxis and/or improvement of a decrease in basal body temperature. When the body temperature is low, the metabolic activity becomes weak, and energy production and body protein synthesis are depressed. Particularly, in elderly people, since thermoregulatory competence is also attenuated, and low body temperature cannot be raised with ease. The nutritional composition of the present invention is useful for the prophylaxis and/or improvement of a decrease in the basal body temperature.

The nutritional composition of the present invention can add lysine and n-3 fatty acid, and
branched chain amino acids in free form
consisting of valine, leucine and isoleucine, in a powder form and the like, to a multipurpose type comprehensive nutritional composition.

The nutritional composition of the present disclosure can be used for increasing muscle mass and/or suppressing a decrease in muscle mass of a person with a decreased muscle mass (e.g., elderly people, bedridden person etc.). It is particularly effective when visceral fat deeply involved in dyslipidemia, hyperglycemia, fatty liver and deterioration of liver function is particularly used as an energy source of muscle protein synthesis. The nutritional composition of the present disclosure greatly contributes to those people having no effective nutritional composition heretofore.

### Examples

The present invention is now explained based on the Examples.

### [Example 1] Preparation of nutritional composition

As a result of mature consideration of an optimum nutritional effect for elderly people, the nutritional composition shown in Table 1 was prepared. The amounts of the starting materials of the liquid for realization thereof are shown in Table 2. Each component and an emulsifier were added to water, mixed, and an emulsion step was repeatedly performed plural times in a high-pressure emulsifying machine under pressurization of 500 - 1,000 kg/cm² to give an emulsion composition. As the amino acid composition, L-leucine (0.38 g), L-valine (0.19 g), L-isoleucine (0.23 g) and L-lysine (0.32 g) were added per 100 kcal of the composition. In this case, the total amount of the branched chain amino acids (leucine, valine, isoleucine) including those derived from a protein is 1.46 g per 100 kcal of the composition. The emulsion composition was filled in an aluminum bag by a conventional filling machine, and sterilized by a retort sterilization machine under general conditions. The nutritional composition of this Example stably contained all ingredients one year later, and the viscosity at 25°C was 9 mPa·s. A thickener was added to the regulated liquid, whereby a nutritional composition with regulated thickness (1,000 - 7,000 mPa·s) or jelly could be produced. Furthermore, even when the amount of the amino acid added was 2-fold, similar production was possible. The calorie density of the nutritional composition was 1.0 kcal/ml.

**Table 1**

| Example 1 nutritional composition | | | |
|---|---|---|---|
| component | | unit | content/100 kcal |
| Protein source | | g | 4.5 |
| | L-leucine | g | 0.38 |
| | L-valine | g | 0.19 |
| | L-isoleucine | g | 0.23 |
| | L-lysine | g | 0.32 |
| lipid | | g | 2.80 |
| | medium chain fatty acid | g | 0.54 |
| | containing EPA | mg | 67 |
| | containing n-3 fatty acid | g | 0.195 |
| hydrocarbonate | | g | 14.2 |
| | carbohydrates | g | 13 |
| | dietary fiber | g | 1.2 |
| | sodium | mg | 185 |
| | calcium | mg | 65 |
| | iron | mg | 0.8 |
| | phosphorus | mg | 55 |
| | magnesium | mg | 26 |
| | potassium | mg | 130 |
| | copper | mg | 0.11 |
| | iodine | µg | 13 |
| | manganese | mg | 0.34 |
| | selenium | µg | 2.5 |
| | zinc | mg | 1.65 |
| | chrome | µg | 2.5 |
| | molybdenum | µg | 2.1 |
| | vitamin A | µg RE *1 | 81 |
| | vitamin D | µg | 0.46 |
| | vitamin E | mg α-TE *2 | 2.73 |
| | vitamin K | µg | 6.3 |
| | vitamin B₁ | mg | 0.6 |
| | vitamin B₂ | mg | 0.36 |
| | niacin | mg NE *3 | 1.3 |
| | vitamin B₆ | mg | 0.6 |
| | folic acid | µg | 20 |
| | vitamin B₁₂ | µg | 0.5 |
| | biotin | µg | 4.2 |
| | pantothenic acid | mg | 0.5 |
| | vitamin C | mg | 40.0 |

| | | | |
|---|---|---|---|
| *1: retinol equivalents *2: amount based on α-tocopherol *3: niacin equivalents | | | |

**[Table 2-1]**

| Example 1 starting material composition | | | |
|---|---|---|---|
| ○ Example 1 composition | | | |
| name of starting materials | mixing ratio (w/v%) | amount added | unit |
| casein sodium | 3.8000 | 114 | kg |
| Lysine hydrochloride | 0.4000 | 12.0 | kg |
| leucine | 0.3833 | 11.5 | kg |
| isoleucine | 0.2300 | 6.90 | kg |
| valine | 0.1900 | 5.70 | kg |
| dextrin | 11.07 | 332 | kg |
| sugar | 2.367 | 71.0 | kg |
| edible vegetable oil | 1.387 | 41.6 | kg |
| medium-chain triglyceride | 0.5367 | 16.1 | kg |
| fish oil | 0.2633 | 7.90 | kg |
| citric acid | 0.1593 | 4.78 | kg |
| trisodium citrate | 0.5433 | 16.3 | kg |
| potassium carbonate | 0.2280 | 6.84 | kg |
| magnesium chloride | 0.2207 | 6.62 | kg |
| calcium lactate | 0.2960 | 8.88 | kg |
| glycerolcalcium phosphate | 0.1303 | 3.91 | kg |
| mineral yeast premix *1 | 0.06293 | 1.888 | kg |
| Indigestible dextrin | 1.5070 | 45.2 | kg |
| emulsifier | 0.6606 | 19.8 | kg |
| β-carotene oil | 0.0015 | 45.0 | g |
| vitamin E | 0.0052 | 156 | g |
| vitamin premix *2 | 0.1035 | 3.105 | kg |
| sodium ascorbate | 0.0810 | 2.43 | kg |
| prepared water | 83.08 | 2492 | kg |
| total | 107.7 | 3231 | kg |

**[Table 2-2]**

| *1 mineral yeast premix composition | | |
|---|---|---|
| name of starting materials | amount | unit |
| molybdenum-containing yeast | 13.0 | g |
| water-soluble chrome-containing yeast | 21.0 | g |
| selenium-containing yeast | 37.0 | g |
| manganese-containing yeast | 203 | g |
| copper-containing yeast | 337 | g |
| zinc-containing yeast | 707 | g |
| biotin-containing yeast | 344 | g |
| sodium ferrous citrate | 226 | g |
| total | 1.888 | kg |

**[Table 2-3]**

| *2 vitamin premix composition | | |
|---|---|---|
| name of starting materials | amount | unit |
| folic acid | 0.60 | g |
| vitamin D powder | 8.50 | g |
| vitamin B 12 0.1% powder | 15.0 | g |
| riboflavin 5'-phosphate sodium | 19.1 | g |
| calcium pantothenate | 22.3 | g |
| pyridoxine hydrochloride | 28.6 | g |
| nicotinic acid amide | 38.5 | g |
| thiamine hydrochloride | 40.7 | g |
| vitamin A powder | 45.7 | g |
| vitamin K2 powder | 58.0 | g |
| kelp extract powder | 278 | g |
| sodium erythorbate | 2550 | g |
| total | 3.105 | kg |

### [Example 2] Preparation of integrated nutritional composition

In the same manner as in Example 1, the nutritional composition shown in Table 3 was prepared. In the same manner as in Example 1, each component and an emulsifier were added to water, mixed, and an emulsion step was repeatedly performed several times in a high-pressure emulsifying machine under pressurization of 50 - 500 kg/cm² to give an emulsion composition. As the amino acid composition, L-leucine (0.28 g), L-valine (0.14 g), L-isoleucine (0.14 g) and L-lysine (0.28 g) were added per 100 kcal of the composition. In this case, the total amount of the branched chain amino acids (leucine, valine, isoleucine) including those derived from a protein is 1.30 g per 100 kcal of the composition. The emulsion composition was filled in an aluminum bag by a conventional filling machine, and sterilized by a retort sterilization machine under general conditions. The nutritional composition of this Example stably contained all ingredients one year later, and the viscosity at 25°C was 4,000 mPa·s. An appropriate amount of a thickener was added to the regulated liquid to further impart viscosity, whereby a nutritional composition with regulated thickness (1,000 - 7,000 mPa·s) or jelly could be produced. The calorie density of the nutritional composition was 2.0 kcal/ml.

**Table 3**

| Example 2 nutritional composition | | | |
|---|---|---|---|
| component | | unit | content/100 kcal |
| Protein source | | g | 4.5 |
| | L-leucine | g | 0.28 |
| | L-valine | g | 0.14 |
| | L-isoleucine | g | 0.14 |
| | L-lysine | g | 0.28 |
| lipid | | g | 2.80 |
| | medium chain fatty acid | g | 0.60 |
| | containing EPA | mg | 67 |
| | containing n-3 fatty acid | g | 0.19 |
| hydrocarbonate | | g | 14.2 |
| | carbohydrates | g | 13 |
| | dietary fiber | g | 1.2 |
| | sodium | mg | 185 |
| | calcium | mg | 65 |
| | iron | mg | 0.8 |
| | phosphorus | mg | 55 |
| | magnesium | mg | 26 |
| | potassium | mg | 130 |
| | copper | mg | 0.11 |
| | iodine | µg | 13 |
| | manganese | mg | 0.34 |
| | selenium | µg | 2.5 |
| | zinc | mg | 1.65 |
| | chrome | µg | 2.5 |
| | molybdenum | µg | 2.1 |
| | vitamin A | µg RE *1 | 81 |
| | vitamin D | µg | 0.46 |
| | vitamin E | mg α-TE *2 | 2.73 |
| | vitamin K | µg | 6.3 |
| | vitamin B₁ | mg | 0.6 |
| | vitamin B₂ | mg | 0.36 |
| | niacin | mg NE *3 | 1.3 |
| | vitamin B₆ | mg | 0.6 |
| | folic acid | µg | 20 |
| | vitamin B₁₂ | µg | 0.5 |
| | biotin | µg | 4.2 |
| | pantothenic acid | mg | 0.5 |
| | vitamin C | mg | 40.0 |

| | | | |
|---|---|---|---|
| *1: retinol equivalents *2: amount based on α-tocopherol *3: niacin equivalents | | | |

### [Example 3] Preparation of powder nutritional composition

The powder type nutritional composition shown in Table 4 was prepared. The amounts of the starting materials of the liquid for realization thereof are shown in Table 5. Each component and an emulsifier were added to an appropriate amount of water, mixed, and an emulsion step was repeatedly performed several times in a high-pressure emulsifying machine under pressurization of 2 - 50 kg/cm² to give an emulsion composition. This emulsion composition was spray dried to give a powder nutritional composition. As the amino acid composition, L-leucine (3.9 g), L-valine (2.4 g), L-isoleucine (2.0 g) and L-lysine (4.1 g) were added per 100 kcal of the composition. This powder nutritional composition was filled in an aluminum bag by a conventional filling machine. The powder nutritional composition of this Example stably contained all ingredients one year later. This nutritional composition could be used even after dissolving in warm water and mixing with a nutritional supplement.

**[Table 4]**

| Example 3 nutritional composition | | | |
|---|---|---|---|
| component | | unit | content/100 kcal |
| Protein source | | g | 12.4 |
| | L-leucine | g | 3.9 |
| | L-valine | g | 2.4 |
| | L-isoleucine | g | 2.0 |
| | L-lysine | g | 4.1 |
| lipid | | g | 4.8 |
| | containing EPA | mg | 812 |
| | containing n-3 fatty acid | g | 1.34 |
| hydrocarbonate | | g | 1.6 |
| | vitamin A | µg RE *1 | 413 |
| | vitamin E | mg α-TE *2 | 19.2 |
| | vitamin B₁ | mg | 5.3 |
| | vitamin B₂ | mg | 3.2 |
| | vitamin B₆ | mg | 5.1 |
| | vitamin C | mg | 392.9 |

| | | | |
|---|---|---|---|
| *1: retinol equivalents *2: amount based on α-tocopherol | | | |

**[Table 5]**

| Example 3 starting material composition | |
|---|---|
| name of starting materials | mixing ratio w/v% |
| L-leucine | 18.65 |
| L-isoleucine | 11.30 |
| L-valine | 9.475 |
| L-lysine hydrochloride | 24.64 |
| fish oil | 22.99 |
| vitamin A oil 500,000 IU/g | 0.01230 |
| thiamine hydrochloride | 0.02571 |
| riboflavin 5'-phosphate sodium | 0.02037 |
| Pyridoxine hydrochloride | 0.03038 |
| tocopherol 40% | 0.2146 |
| L ascorbic acid Na | 2.146 |
| emulsifier | 10.487 |
| | 100.0 |

### [Experimental Example 1] Study of enrichment effect of amino acid and n-3 fatty acid in visceral fat increase animal model

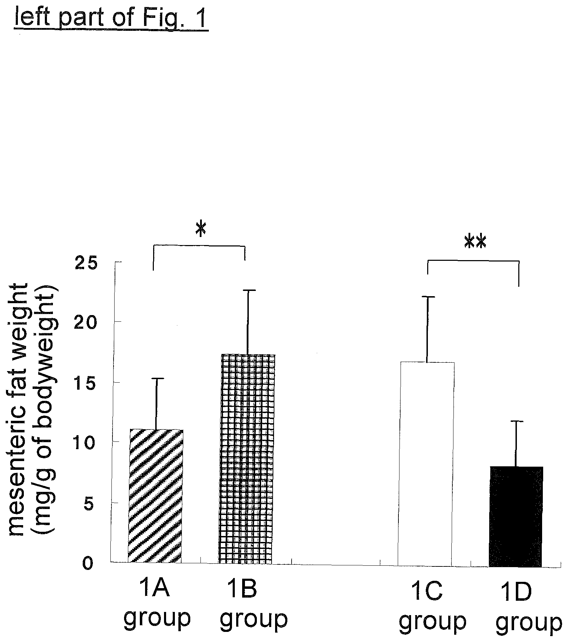

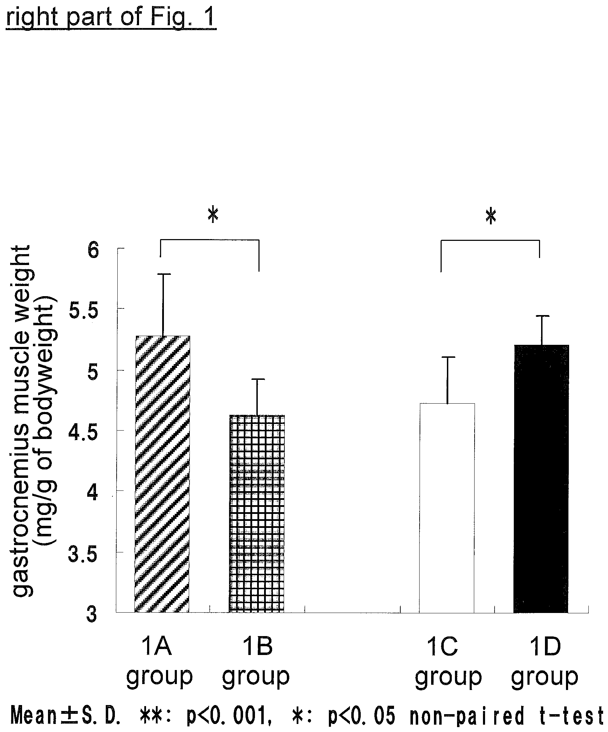

Improving effects of amino acid and n-3 fatty acid in the nutritional composition of the present invention on visceral fat increase and muscle mass decrease was examined by the following experiment. To be specific, 10-week-old male C57BL/6J mice were fed with the same high-fat diet as in 1B group shown in Table 6 to prepare visceral fat increase models. They were divided into 3 groups (each group N=5-9), and fed with an experimental diet of the composition shown in Table 6 for 2 weeks. 1B group was continuously fed with a high-fat diet containing a large amount of beef fat rich in saturated fatty acid. 1C group was fed with an experimental diet of a standard composition. 1D group was fed with an experimental diet with an enriched composition, wherein the total amount of protein and fat was not changed, and 0.32 g, 0.8 g, 0.24 g of lysine, BCAA (3 kinds of branched chain amino acid consisting of leucine, valine, isoleucine) and fish oil, respectively, per 100 kcal of experimental diet, were used for substitution. In this case, 1D group was fed with 1.46 g of the total amount of BCAA including those derived from protein, 0.24 g of the total amount of n-3 fatty acid and 67 mg of eicosapentaenoic acid (EPA), each per 100 kcal of the experimental diet. 1A group as a normal control group was fed with an experimental diet with a standard composition throughout the test period, without a high-fat diet. Two weeks after ingestion of experimental diets, they were fasted for 16 hr, and the mesenteric fat weight and gastrocnemius muscle weight were measured. The results are shown in Fig. 1.

**[Table 6]**

| experimental diet composition table (content per 100 kcal of experimental diet) | | | | | | | | | | |
|---|---|---|---|---|---|---|---|---|---|---|
| group constitution | Protein source (g) | | | | | fat (g) | | | | hydrocarbonate (g) |
| | casein (g) | L-lysine (g) | L-valine (g) | L-leucine (g) | L-isoleucine (g) | soybean oil (g) | beef fat (g) | fish oil (g) containing EPA (mg) | n-3 fatty acid amount contained (g) | |
| 1A group | 4.5 | | | | | 2.80 | | | 0.17 | 15.4 |
| 1B group | 4.5 | | | | | 0.56 | 5.17 | | 0.05 | 7.2 |
| | | | | | | | | | | |
| 1C group | 4.5 | | | | | 2.80 | | | 0.17 | 15.4 |
| 1D group | 3.38 | 0.32 | 0.2 | 0.4 | 0.2 | 2.56 | | 0.24 67 | 0.24 | 15.4 |

The body weight of 1B group fed with a high-fat diet was 1.2 times that of 1A group fed with a general diet. Thereafter, throughout the evaluation period when the experimental diet was given, the body weight of 1D group was lower than that of 1C group, even though the calorie intake was the same (1D: 31.7±3.3 g, 1C: 34.4±1.7 g). Moreover, as shown in Fig. 1, the mesenteric fat weight significantly increased in 1B group as compared to 1A group, and the gastrocnemius muscle weight significantly decrease in 1B group as compared to 1A group. On the other hand, the mesenteric fat weight significantly decreased in 1D group and the gastrocnemius muscle weight significantly increased, as compared to 1C group. In this high-fat diet ingestion model, CRP in plasma (C reactive protein) showed no difference between groups (1A group: 7.7±2.4 ng/ml, 1B group: 9.0± 3.3 ng/ml, 1C group: 7.0±5.3 ng/ml, 1D group: 8.6±3.9 ng/ml).

From the above-mentioned test results, it has been found that a diet enriched with lysine, BCAA, eicosapentaenoic acid can decrease mesenteric fat weight, which is the weight of visceral fat, and increase gastrocnemius muscle weight.

### [Experimental Example 2] Single effect and combined use effect of amino acids (lysine, BCAA) and n-3 fatty acid used in the present invention

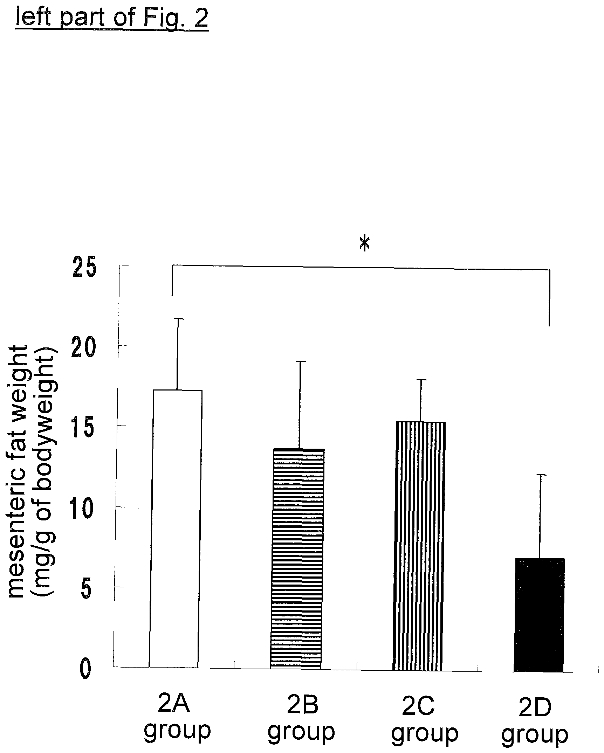

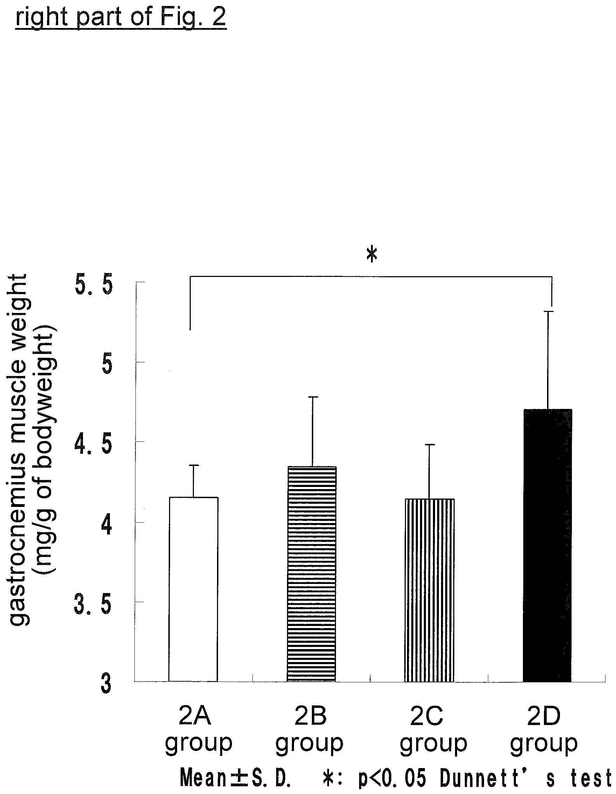

As a result of Experimental Example 1, it has been clarified that enrichment with amino acid and n-3 fatty acid used in the present invention is necessary for showing mesenteric fat weight decreasing and gastrocnemius muscle weight increasing actions. However, it is not clear whether each component shows such effect by itself. Therefore, the following was studied wherein amino acid and n-3 fatty acid used in the present invention were singly or combinedly used for enrichment. That is, visceral fat increase models were prepared in the same manner as in Experimental Example 1, divided into 4 groups (each group N=5-9), and fed with an experimental diet of the composition shown in Table 7 for 2 weeks. 2A group was fed with an experimental diet of a standard composition. 2D group was fed with an experimental diet with an enriched composition, wherein the total amount of protein and fat was not changed, and 0.32 g, 0.8 g, 0.24 g of lysine, BCAA and fish oil, respectively, per 100 kcal of experimental diet, were used for substitution. In this case, 2D group was fed with 1.46 g of the total amount of BCAA including those derived from protein, 0.24 g of the total amount of n-3 fatty acid and 67 mg of eicosapentaenoic acid (EPA), each per 100 kcal of the experimental diet. Furthermore, as groups fed with an experimental diet enrich with each of amino acid, n-3 fatty acid used in the present invention, 2B group, 2C group were formed. Two weeks after ingestion of each experimental diet, they were fasted for 16 hr, and the mesenteric fat weight and gastrocnemius muscle weight were measured. The results are shown in Fig. 2.

**[Table 7]**

| experimental diet composition table (content per 100 kcal of experimental diet) | | | | | | | | | |
|---|---|---|---|---|---|---|---|---|---|
| group constitution | Protein source (g) | | | | | fat (g) | | | hydrocarbonate (g) |
| | casein (g) | L-lysine (g) | L-valine (g) | L-leucine (g) | L-isoleucine (g) | soybean oil (g) | fish oil (g) containing EPA (mg) | n3 fatty acid amount contained (g) | |
| 2A group | 4.5 | | | | | 2.80 | | 0.17 | 15.4 |
| 2B group | 3.38 | 0.32 | 0.2 | 0.4 | 0.2 | 2.80 | | 0.17 | 15.4 |
| 2C group | 4.5 | | | | | 2.56 | 0.24 | 0.24 | 15.4 |
| | | | | | | | 67 | | |
| 2D group | 3.38 | 0.32 | 0.2 | 0.4 | 0.2 | 2.56 | 0.24 | 0.24 | 15.4 |
| | | | | | | | 67 | | |

The total calorie intake showed no difference between groups in the experimental diet ingestion period. As shown in Fig. 2, 2D group significantly decreased mesenteric fat weight, which is the visceral fat, and significantly increased gastrocnemius muscle weight. In contrast, decrease of mesenteric fat weight and increase of gastrocnemius muscle weight in 2B group, 2C group were clearly weak as compared to the effects in 2D group, and the effect in 2B group added with the effect in 2C group was still lower than the effect in 2D group.

From the above-mentioned results, it is considered that the mesenteric fat decreasing action and gastrocnemius muscle increasing action by the enriched diet was not provided by the amino acid or n-3 fatty acid alone used in the present invention, but by a synergistic effect afforded by the amino acid and n-3 fatty acid in combination. That is, in this test, the usefulness of combined use of lysine and n-3 fatty acid, and further, branched chain amino acid was shown.

### [Experimental Example 3] Energy metabolism enhancing effect and mitochondria increasing effect of amino acids and n-3 fatty acid used in the present invention

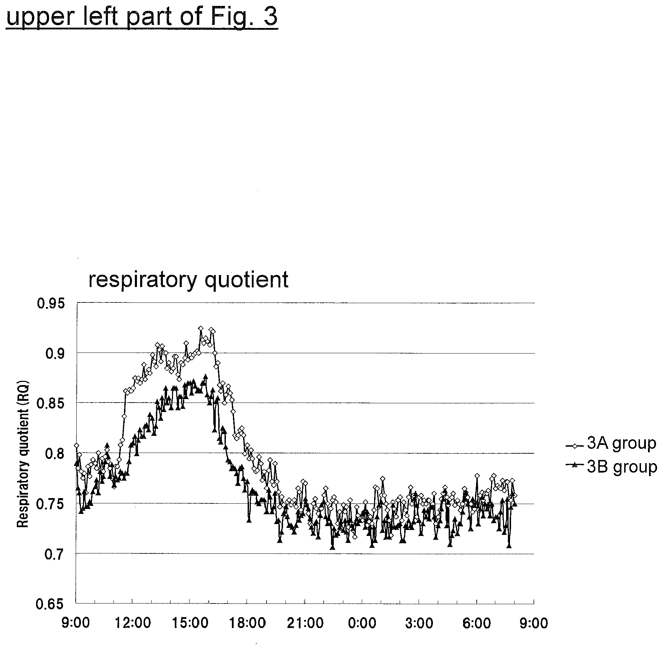

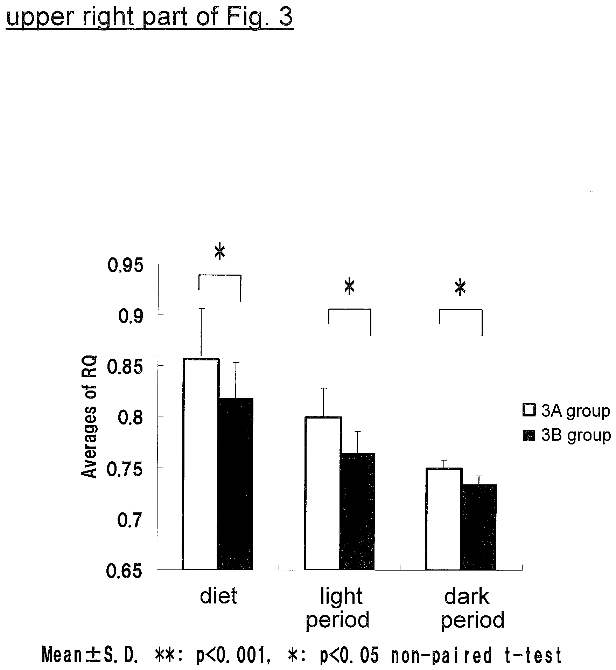

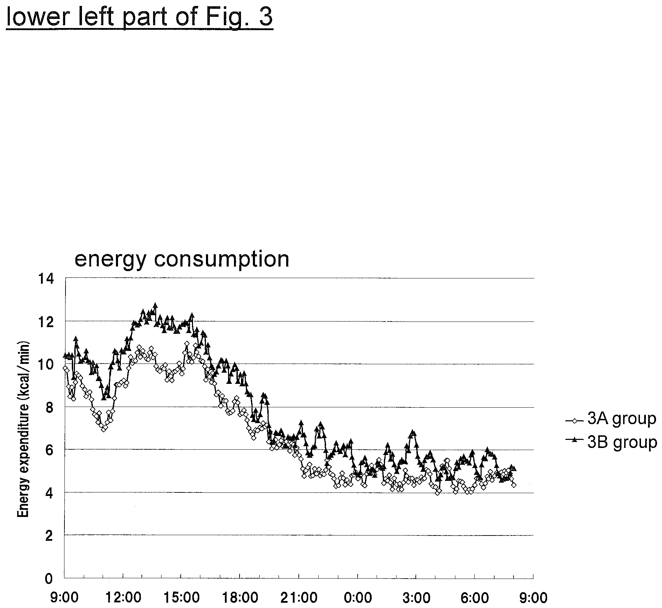

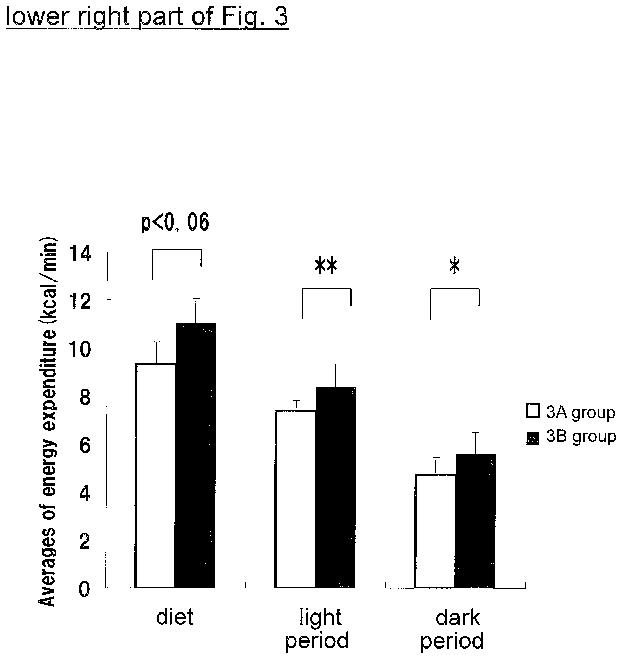

Then, an influence of the nutritional composition enriched with amino acid and n-3 fatty acid of the present invention on the measurement of energy metabolism using an exhaled gas metabolism measuring apparatus was examined. That is, visceral fat increase model prepared in the same manner as in Experimental Example 1 was fed with an experimental diet with a standard composition (3A group) or an experimental diet with an enriched composition (3B group) shown in Table 8, and subjected to an evaluation. To stabilize the amount of intake, a one-week experimental diet acclimation period was set, after which one animal was placed in each cage within 2 weeks, and oxygen consumption (ml/min) and carbon dioxide production (ml/min) were measured (metabolism measuring system for small animals MK-5000 RQ, Muromachi Kikai Co., Ltd.). During the measurement, an experimental diet was given, and the amount of intake was measured. The respiratory quotient was calculated by carbon dioxide production ÷ oxygen consumption, and the energy consumption was calculated by the formula of Weir [energy consumption (kcal/min)=3.9 (kcal)×oxygen consumption (ml/min) + 1.1 (kcal) × carbon dioxide production (ml/min)]. The results are shown in Fig. 3.

**[Table 8]**

| | | | | | | | | | |
|---|---|---|---|---|---|---|---|---|---|
| experimental diet composition table (content per 100 kcal of experimental diet) | | | | | | | | | |

| group constitution | Protein source (g) | | | | | fat (g) | | | hydrocarbonate (g) |
|---|---|---|---|---|---|---|---|---|---|
| | casein (g) | L-lysine (g) | L-valine (g) | L-leucine (g) | L-isoleucine (g) | soybean oil (g) | fish oil (g) containing EPA (mg) | n3 fatty acid amount (g) contained | |
| 3A group | 4.5 | | | | | 2.80 | | 0.17 | 15.4 |
| 3B group | 3.38 | 0.32 | 0.2 | 0.4 | 0.2 | 2.56 | 0.24 | 0.24 | 15.4 |
| | | | | | | | 67 | | |

As shown in Fig. 3, the respiratory quotient of 3B group significantly decreased than in 3A group during diet ingestion period (9:00 - 16:00; diet) and dark period after eating (16:00 - 19:00; dark period), and significantly decreased than in 3A group even in the light period (19:00 - 9:00: light period) which is free of a direct influence of eating. The energy consumption of 3B group tends to significantly increase or increase than in 3A group at any period.

In addition, a mitochondria increasing effect of the nutritional composition of the present invention was examined. That is, visceral fat increase model prepared in the same manner as in Experimental Example 1 was fed with an experimental diet with a standard composition (3A group) or an experimental diet with an enriched composition (3B group) shown in Table 8, and, on the last day of the test, the liver and gastrocnemius muscle were rapidly collected and cryopreserved. DNA was extracted from each of the cryopreserved liver (100 mg) and gastrocnemius muscle (200 mg) by using ISOGEN (Japan gene). To 50 ng DNA were added the primer of d-loop of mitochondria shown in Table 9 and SYBER GREEN Master Mix (ABI), and amplification and quantification analysis were performed using an ABI 7700 detector. For the amendment of DNA amount, gene cyclophillin A (intron code part) on the chromosome was used. As for the synthesized primer, the occurrence of desired production was confirmed by agarose electrophoresis after PCR reaction.

**[Table 9]**

| DNA primer of mitochondria and chromosome | | |
|---|---|---|
| Gene | sense | antisense |
| ch cyclophillin A | ACACGCCATAATGGCACTGG (Sequence Listing SEQ ID NO: 1) | CAGTCTTGGCAGTGCAGAT (Sequence Listing SEQ ID NO: 2) |
| mt d-loop | CGCAAAACCCAATCACCTAA (Sequence Listing SEQ ID NO: 3) | TTGGGGTTTGGCATTAAGAG (Sequence Listing SEQ ID NO: 4) |

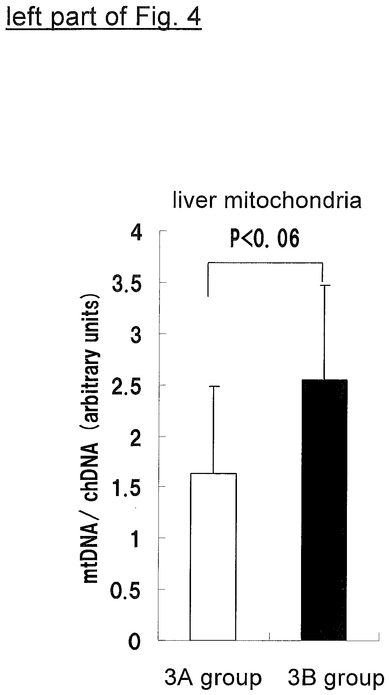

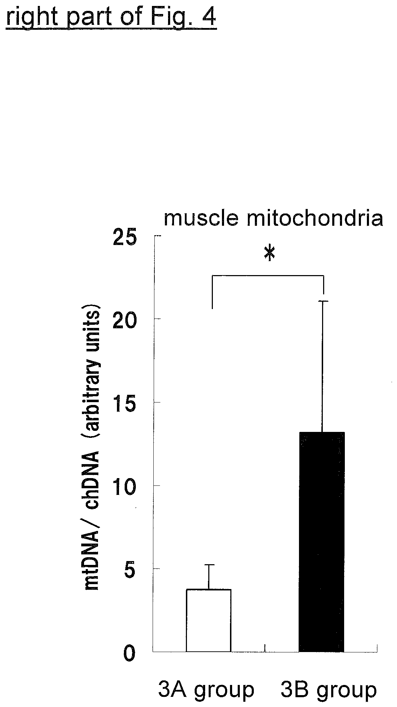

Since mitochondria plays an important role in fat combustion in the cell, the number of intracellular mitochondria was analyzed. While only one intranuclear chromosomal DNA is present in the cell, the number of mitochondria is not the same. Therefore, the amount of mitochondria in the cell was assumed by a method of determining the ratio of the DNA amount of mitochondrial coding gene and that of the nuclear coding gene. The results are shown in Fig. 4.

As shown in Fig. 4, the mitochondrial DNA amount of 3B group significantly increased in both the liver and gastrocnemius muscle, as compared to 3A group.

By this test, it has been suggested that a nutritional composition containing lysine and n-3 fatty acid, and further, a branched chain amino acid increases the number of mitochondria in a fat utilizing site to enhance fat utilization, and can increase energy consumption that leads to an increase in the body temperature and the like.

### Industrial Applicability

The nutritional composition of the present disclosure is effective for increasing muscle mass and/or suppressing a decrease in muscle mass. The nutritional composition of the present invention is effective for increasing energy consumption. The nutritional composition of the present invention is particularly useful for decreasing visceral fat by increasing energy consumption by enhanced fat utilization and the like. The composition is used
for the prophylaxis and/or improvement of dyslipidemia, hyperglycemia, fatty liver, and deterioration of liver function associated with visceral fat increase. Furthermore, by increasing muscle mass or suppressing a decrease in muscle mass by fat energy supply and the like, the nutritional composition of the present disclosure is also useful for the prophylaxis and/or improvement of sarcopenia, chronic obstructive pulmonary disease (COPD), promotion of rehabilitation effect for muscle recovery, improvement of low nutrient condition, improvement of deterioration in locomotorium, prophylaxis and/or improvement of locomotive syndrome, prevention of falling, increasing muscle mass in sports and the like. Moreover, the nutritional composition of the present invention is effective for the prophylaxis and/or improvement of a decrease in basal energy consumption, and prophylaxis and/or improvement of a decrease in basal body temperature. Furthermore, the nutritional composition of the present invention is highly safe, and can be used for a long time without placing an excessive protein load even for elderly people with attenuated kidney function.

## Claims

1. A nutritional composition for use in the prophylaxis and/or improvement of a condition by decreasing visceral fat or increasing energy consumption, the composition comprising: n-3 fatty acid; one or more kinds selected from the group consisting of free lysine, dipeptides containing lysine and lysine salts; and branched chain amino acids in free form consisting of valine, leucine and isoleucine; wherein
the content of the total amount of one or more kinds selected from the group consisting of free lysine, dipeptides containing lysine and lysine salts is 0.1 g - 10.0 g per 100 kcal of the composition, and
the content of the total amount of n-3 fatty acid is 0.17 g - 5.00 g per 100 kcal of the composition, and wherein the n-3 fatty acid comprises eicosapentaenoic acid; wherein the condition is at least one of:
dyslipidemia associated with visceral fat increase;
hyperglycemia associated with visceral fat increase;
fatty liver associated with visceral fat increase;
deteriorated liver function associated with visceral fat increase;
decrease in basal energy consumption; and
decrease in basal body temperature.

2. A nutritional composition for use according to claim 1, wherein the condition is at least one of: dyslipidemia associated with visceral fat increase; hyperglycemia associated with visceral fat increase; fatty liver associated with visceral fat increase; and deteriorated liver function associated with visceral fat increase;.

3. A nutritional composition for use according to claim 1, wherein the condition is at least one of decrease in basal energy consumption and
decrease in basal body temperature.

4. The nutritional composition for use according to any one of claims 1 to 3, wherein the content of the total amount of one or more kinds of branched chain amino acids in a free form selected from the group consisting of valine, leucine and isoleucine is 0.1 g - 15 g per 100 kcal of the composition.

5. The nutritional composition for use according to any one of the preceding claims, wherein the n-3 fatty acid further comprises one or more kinds selected from the group consisting of docosapentaenoic acid and docosahexaenoic acid.

6. The nutritional composition for use according to any one of the preceding claims, comprising 10 mg - 3000 mg of eicosapentaenoic acid per 100 kcal of the composition.

7. The nutritional composition for use according to any one of the preceding claims, comprising one or more kinds selected from the group consisting of
0.2 mg - 20.0 mg of vitamin B1 per 100 kcal of the composition,
0.25 mg - 20.0 mg of vitamin B2 per 100 kcal of the composition,
0.3 mg - 20.0 mg of vitamin B6 per 100 kcal of the composition, and
0.3 µg - 20.0 µg of vitamin B12 per 100 kcal of the composition.

8. The nutritional composition for use according to any one of the preceding claims, comprising one or more kinds selected from the group consisting of
50 µg retinol equivalents - 2500 µg retinol equivalents of vitamin A per 100 kcal of the composition,
10 mg - 700 mg of vitamin C per 100 kcal of the composition, and
1 mg - 50 mg of vitamin E based on α-tocopherol per 100 kcal of the composition.

9. The nutritional composition for use according to any one of the preceding claims, comprising 1 mg - 50 mg of zinc per 100 kcal of the composition and/or 1.0 µg - 50.0 µg of selenium per 100 kcal of the composition.

10. The nutritional composition for use according to any one of the preceding claims, comprising medium chain fatty acid oil at a content of 10 wt% - 65 wt% relative to the total amount of lipid.

## Patentansprüche

1. Nährstoffzusammensetzung zur Verwendung bei der Prophylaxe und/oder Verbesserung eines Zustandes durch Verringerung des viszeralen Fetts oder Erhöhung des Energieverbrauchs, wobei die Zusammensetzung umfasst: n-3 Fettsäure; eine oder mehrere Arten, ausgewählt aus der Gruppe bestehend aus freiem Lysin, Dipeptiden, die Lysin und Lysinsalze enthalten; und verzweigtkettige Aminosäuren in freier Form bestehend aus Valin, Leucin und Isoleucin;
worin der Gehalt der Gesamtmenge einer oder mehrerer Arten, ausgewählt aus der Gruppe bestehend aus freiem Lysin, lysinhaltigen Dipeptiden und Lysinsalzen, 0,1 g - 10,0 g pro 100 kcal der Zusammensetzung ist, und
der Gehalt der Gesamtmenge an n-3 Fettsäure 0,17 g - 5,00 g pro 100 kcal der Zusammensetzung ist, und worin die n-3-Fettsäure Eicosapentaensäure umfasst; wobei der Zustand mindestens einer der folgenden ist:
Dyslipidämie im Zusammenhang mit der Zunahme von viszeralem Fett;
Hyperglykämie im Zusammenhang mit dem Anstieg des viszeralen Fettgehalts;
Fettleber im Zusammenhang mit dem Anstieg des viszeralen Fettgehalts;
verschlechterte Leberfunktion im Zusammenhang mit dem Anstieg des viszeralen Fettgehalts;
Rückgang des basalen Energieverbrauchs; und
Abnahme der Basaltemperatur.

2. Nährstoffzusammensetzung zur Verwendung nach Anspruch 1, worin der Zustand mindestens einer ist, der unter Dyslipidämie im Zusammenhang mit der Erhöhung des viszeralen Fettgehalts; Hyperglykämie im Zusammenhang mit dem Anstieg des viszeralen Fettgehalts; Fettleber im Zusammenhang mit dem Anstieg des viszeralen Fettgehalts; und verschlechterter Leberfunktion im Zusammenhang mit dem Anstieg des viszeralen Fettgehalts ausgewählt ist.

3. Nährstoffzusammensetzung zur Verwendung nach Anspruch 1, wobei der Zustand mindestens einer ist, der unter Abnahme des Basalenergieverbrauchs und Abnahme der Basaltemperatur ausgewählt ist.

4. Nährstoffzusammensetzung zur Verwendung nach einem der Ansprüche 1 bis 3, worin der Gehalt der Gesamtmenge einer oder mehrerer Arten verzweigtkettiger Aminosäuren in freier Form, die aus der aus Valin, Leucin und Isoleucin bestehenden Gruppe ausgewählt sind, 0,1 g 15 g pro 100 kcal der Zusammensetzung beträgt.

5. Nährstoffzusammensetzung zur Verwendung nach einem der vorstehenden Ansprüche, worin die n-3-Fettsäure ferner eine oder mehrere Arten umfasst, die aus der Gruppe ausgewählt sind, die aus Docosapentaensäure und Docosahexaensäure besteht.

6. Nährstoffzusammensetzung zur Verwendung nach einem der vorstehenden Ansprüche, umfassend 10 mg 3000 mg Eicosapentaensäure pro 100 kcal der Zusammensetzung.

7. Nährstoffzusammensetzung zur Verwendung nach einem der vorstehenden Ansprüche, umfassend eine oder mehrere Arten, ausgewählt aus der Gruppe bestehend aus
0,2 mg - 20,0 mg Vitamin B1 pro 100 kcal der Zusammensetzung,
0,25 mg - 20,0 mg Vitamin B2 pro 100 kcal der Zusammensetzung,
0,3 mg - 20,0 mg Vitamin B6 pro 100 kcal der Zusammensetzung und
0,3 µg - 20,0 µg Vitamin B12 pro 100 kcal der Zusammensetzung.

8. Nährstoffzusammensetzung zur Verwendung nach einem beliebigen Verfahren der vorhergehenden Ansprüche, umfassend eine oder mehrere Arten, ausgewählt aus der Gruppe bestehend aus
50 µg Retinoläquivalente - 2500 µg Retinoläquivalente von Vitamin A pro 100 kcal der Zusammensetzung,
10 mg - 700 mg Vitamin C pro 100 kcal der Zusammensetzung, und
1 mg - 50 mg Vitamin E auf Basis von α-Tocopherol pro 100 kcal der Zusammensetzung.

9. Nährstoffzusammensetzung zur Verwendung nach einem beliebigen Verfahren der vorhergehenden Ansprüche, umfassend 1 mg - 50 mg Zink pro 100 kcal der Zusammensetzung und/oder 1,0 µg - 50.0 µg Selen pro 100 kcal der Zusammensetzung.

10. Nährstoffzusammensetzung zur Verwendung gemäß einem der vorhergehenden Ansprüche, umfassend Fettsäureöl mittlerer Kettenlänge mit einem auf die Gesamtmenge an Lipid bezogenen Gehalt von 10 Gew.% - 65 Gew.%.

## Revendications

1. Composition nutritionnelle pour son utilisation dans la prophylaxie et/ou l'amélioration d'une affection par réduction de la graisse viscérale ou augmentation de la consommation d'énergie, ladite composition comprenant : un acide gras n-3 ; un ou plusieurs éléments sélectionnés dans le groupe consistant en la lysine libre, les dipeptides contenant de la lysine et les sels de lysine ; et les acides aminés à chaîne ramifiée sous forme libre consistant en la valine, la leucine et l'isoleucine ; dans laquelle
la teneur de la quantité totale d'un ou plusieurs éléments sélectionnés dans le groupe consistant en la lysine libre, les dipeptides contenant de la lysine et les sels de lysine est de 0,1 g à 10,0 g pour 100 kcal de la composition, et
la teneur de la quantité totale en acide gras n-3 est de 0,17 g à 5,00 g pour 100 kcal de la composition, et dans laquelle l'acide gras n3 comprend de l'acide éicosapentaénoïque ; dans laquelle l'affection est au moins l'une choisie parmi :
la dyslipidémie associée à une augmentation de la graisse viscérale ;
l'hyperglycémie associée à une augmentation de la graisse viscérale ;
la stéatose hépatique associée à une augmentation de la graisse viscérale ;
une altération de la fonction hépatique associée à une augmentation de la graisse viscérale ;
une réduction de la consommation d'énergie basale ; et
une réduction de la température corporelle basale.

2. Composition nutritionnelle pour son utilisation selon la revendication 1, dans laquelle l'affection est au moins l'une choisie parmi : la dyslipidémie associée à une augmentation de la graisse viscérale ; l'hyperglycémie associée à une augmentation de la graisse viscérale ; la stéatose hépatique associée à une augmentation de la graisse viscérale ; et une altération de la fonction hépatique associée à une augmentation de la graisse viscérale.

3. Composition nutritionnelle pour son utilisation selon la revendication 1, dans laquelle l'affection est au moins l'une choisie parmi la réduction de la consommation d'énergie basale et
la réduction de la température corporelle basale.

4. Composition nutritionnelle pour son utilisation selon l'une quelconque des revendications 1 à 3, dans laquelle la teneur de la quantité totale d'un ou plusieurs acides aminés à chaîne ramifiée sous forme libre sélectionnés dans le groupe consistant en la valine, la leucine et l'isoleucine est de 0,1 g à 15 g pour 100 kcal de la composition.

5. Composition nutritionnelle pour son utilisation selon l'une quelconque des revendications précédentes, dans laquelle l'acide gras n-3 comprend en outre un ou plusieurs sélectionnés dans le groupe consistant en l'acide docosapentaénoïque et l'acide docosahexaénoïque.

6. Composition nutritionnelle pour son utilisation selon l'une quelconque des revendications précédentes, comprenant de 10 mg à 3 000 mg d'acide éicosapentaénoïque pour 100 kcal de la composition.

7. Composition nutritionnelle pour son utilisation selon l'une quelconque des revendications précédentes, comprenant un ou plusieurs éléments sélectionnés dans le groupe consistant en
de 0,2 mg à 20,0 mg de vitamine B1 pour 100 kcal de la composition,
de 0,25 mg à 20,0 mg de vitamine B2 pour 100 kcal de la composition,
de 0,3 mg à 20,0 mg de vitamine B6 pour 100 kcal de la composition, et
de 0,3 µg à 20,0 µg de vitamine B12 pour 100 kcal de la composition.

8. Composition nutritionnelle pour son utilisation selon l'une quelconque des revendications précédentes, comprenant un ou plusieurs éléments sélectionnés dans le groupe consistant en
de 50 µg à 2 500 µg d'équivalents rétinol de vitamine A pour 100 kcal de la composition,
de 10 mg à 700 mg de vitamine C pour 100 kcal de la composition, et
de 1 mg à 50 mg de vitamine E sur la base de l'a-tocophérol pour 100 kcal de la composition.

9. Composition nutritionnelle pour son utilisation selon l'une quelconque des revendications précédentes, comprenant de 1 mg à 50 mg de zinc pour 100 kcal de la composition et/ou de 1,0 µg à 50,0 µg de sélénium pour 100 kcal de la composition.

10. Composition nutritionnelle pour son utilisation selon l'une quelconque des revendications précédentes, comprenant une huile d'acide gras à chaîne moyenne à une teneur allant de 10 % en poids à 65 % en poids par rapport à la quantité totale de lipide.
